# EUROPÄISCHE PATENTANMELDUNG

(11) **EP 3 348 646 A1**
(43) Veröffentlichungstag der Anmeldung: **18.07.2018**
(21) Anmeldenummer: 17151723.8
(22) Anmeldetag: 17.01.2017
(51) Int. Cl.: C12P 7/04, C12P 7/06, C12P 7/16, C12P 7/28, C12P 7/30, C07C 29/76, C07C 45/78

(54) **MIKROBIELLES VERFAHREN ZUR HERSTELLUNG VON ACETON, ISOPROPANOL, BUTANOL UND/ODER ETHANOL UMFASSEND DIE PRODUKTABSORPTION DURCH WASSER**

(71) Anmelder: Evonik Degussa GmbH, 45128 Essen (DE)
(72) Erfinder: NITZ, Jörg-Joachim, 45257 Essen (DE); GERDOM, Marzena, 40476 Düsseldorf (DE); KOHLSTRUK, Stephan, 45966 Gladbeck (DE); BLUEMKE, Wilfried, 61137 Schöneck (DE)

(57) **Zusammenfassung**

Gegenstand der Erfindung ist ein Verfahren zur Aufarbeitung von organischem Produkt ausgewählt aus der Gruppe Aceton, Isopropanol, Butanol und Ethanol aus einem Fermentationsprozess mit Wasser als Absorptionsmittel.

## Beschreibung

### Gebiet der Erfindung

Gegenstand der Erfindung ist ein Verfahren zur Aufarbeitung von organischem Produkt ausgewählt aus der Gruppe Aceton, Isopropanol, Butanol und Ethanol aus einem Fermentationsprozess mit Wasser als Absorptionsmittel.

### Stand der Technik

Das Lösungsmittel Aceton ist eine wichtige Plattformchemikalie der Chemischen Industrie mit stetig steigendem Bedarf.

Es besteht ein großes Interesse alternative Herstellverfahren zur Herstellung von Aceton auf der Basis zukunftsträchtiger Kohlenstoffquellen, wie bspw. aus nachwachsenden Rohstoffen (z. B. Zucker, Getreide, Biomasse -Reststoffe aus Land- und Forstwirtschaft) oder sogar gasförmigen Kohlenstoffquellen (z. B. Kohlenstoffdioxid (CO₂) oder Kohlenstoffmonoxid (CO) enthaltende Gasströme) zu entwickeln. Fermentationsprozesse jeglicher Art (direkte Zucker-Fermentation / Lignocellulose-Hydrolyse Integrierte Fermentation / Syngas-Fermentation) können eine Alternative zur Herstellung der Plattformchemikalie Aceton bieten. Alle fermentativen Herstellungsverfahren haben das große Problem, dass die Trennung des organischen Wertprodukts (Aceton) aus der Fermentationsbrühe zwingend notwendig aber gleichzeitig hochkomplex ist und eine zu hohe Produktkonzentration zu einer Inhibierung führt.

Die klassische Aceton-Butanol-Ethanol- (ABE) Fermentation mit Clostridien (z. B.: *Clostridium acetobutylicum*) wurde bereits im Jahre 1916 industriell genutzt und liefert neben Aceton und Ethanol insbesondere Butanol. Das Aceton-Butanol-Ethanol liegt ungefähr im Verhältnis 3:6:1 in der Fermentationsbrühe vor.

Qureshi und Blaschek beschreiben in Renewable Energy, 22(4): 557-564 und Ezeji et al. in Appl Microbiol Biotechnol, 63(6):653-8 ein ABE-Verfahren, in dem die niedermolekularen, organischen Verbindungen per Gasstripping aus der Fermentationsbrühe ausgetragen werden und in Kühlfallen adsorbiert werden. Dieses Verfahren hat den Nachteil eines hohen Energiebedarfs, da die Kühlung kontinuierlich aufrechterhalten werden muss. Ein weiterer Nachteil ist, dass in den Kühlfallen die Produkte nur unzureichend adsorbiert werden, ein beträchtlicher Anteil somit aus der Falle wieder ausgetragen und gegebenenfalls in den Fermenter zurückgeführt wird, wodurch es zu unnötiger Produktinhibition kommen kann, bzw. wenn keine Rückführung erfolgt es zu einem deutlichen Ausbeuteverlust kommt.

Das Problem einer möglichen Produktinhibierung wird in DE102011077705A1 gelöst durch ein *in* -*situ*-Gasstripping noch während der Fermentation und anschließender Absorption von niedermolekularen, organischen Verbindungen mit Hilfe von Isophoron. Die Wiederfindungsrate der niedermolekularen Verbindungen ist im Vergleich zu einer Adsorption durch Kühlfallen verbessert aber noch nicht optimal. Bei Isophoron handelt es sich um das trimere Kondensationsprodukt des Acetons und um ein hochsiedendes Lösemittel, das u. a. auch in der Lack-, Druckfarben-, Klebstoff- und Pflanzenschutzmittel-Industrie Verwendung findet. Dieses Verfahren hat jedoch die folgenden Nachteile:
- Verwendung eines organisches Lösungsmittels (Isophoron) und somit eines entzündlichen Gefahrstoffs
- Hohe Aufarbeitungskosten durch Verwendung von Isophoron
- Absorptionsvermögen von Isophoron für Aceton nicht optimal, für eine Steigerung des Absorptionsvermögens sind hohe Drücke und somit auch höhere Materialkosten und Betriebskosten notwendig.

Aufgabe der Erfindung war es, ein Verfahren bereitzustellen welches in der Lage ist ressourcen- und umweltschonend eine Aufarbeitung von organischem Produkt ausgewählt aus der Gruppe Aceton, Isopropanol, Butanol und Ethanol aus einer wässrigen Fermentationsbrühe zu ermöglichen.

### Beschreibung der Erfindung

Überraschenderweise wurde gefunden, dass das im Folgenden beschriebene Verfahren umfassend eine Absorption von organischem Produkt ausgewählt aus der Gruppe Aceton, Isopropanol, Butanol und Ethanol mit Hilfe von Wasser (H₂O) die der Erfindung gestellte Aufgabe zu lösen vermag.

Ein Vorteil der vorliegenden Erfindung ist es, dass das erfindungsgemäße Verfahren in den Kilotonnenmaßstab skalierbar ist.

Noch ein Vorteil der vorliegenden Erfindung ist es, dass das erfindungsgemäße Verfahren es ermöglicht, dass das organische Produkt durch Strippen noch während der Fermentation abzutrennen ist.

Ein weiterer Vorteil der vorliegenden Erfindung ist es, dass für das Reaktionsgemisch Wasser/Aceton unter Normaldruck kein Azeotrop vorliegt, so dass in einer abschließenden Destillation eine vollständige Trennung von Wasser und Aceton möglich ist.

Noch ein Vorteil der vorliegenden Erfindung ist es, dass das erfindungsgemäße Verfahren eine Gesamtausbeute von über 95 Gew.-% erreichen kann.

Ein weiterer Vorteil der vorliegenden Erfindung ist es, dass der Verlust des Absorbens (Wasser) gering ist.

Noch ein Vorteil der vorliegenden Erfindung ist es im Fall des Acetons, dass das mit dem erfindungsgemäßen Verfahren hergestellte Aceton die notwendige Spezifikation für die weiteren chemischen Umsetzungen zu kommerziellen Aceton-Folgeprodukten, wie beispielsweise zu Isophoron, Methylmethacrylat oder Bisphenol A, erfüllt.

Ein weiterer Vorteil der vorliegenden Erfindung ist es, dass es sich bei Wasser um keinen Gefahrstoff handelt, so dass dieses Verfahren zusätzlich unter Sicherheits- und Umweltaspekten einen deutlichen Vorteil bietet.

Noch ein Vorteil der vorliegenden Erfindung ist es im Fall des Acetons, dass im Gegensatz zu organischen Lösungsmitteln bei Wasser als Absorbens ein moderater Verlust sowohl aus ökonomischen, als auch aus Umwelt- und Sicherheitstechnischen Aspekten vertretbar ist.

Ein weiterer Vorteil der vorliegenden Erfindung ist es, dass durch den ermöglichten Betrieb bei Normaldruck, bzw. moderaten Drücken es nur eines geringen technischen Aufwands bedarf.

Ein Vorteil der vorliegenden Erfindung ist es, dass das erfindungsgemäße Aufarbeitungsverfahren sowohl auf anaerobe, als auch auf aerobe Fermentationen angewendet werden kann. Das Gasstripping kann also sowohl mit elementar sauerstoffhaltigen Gasen als Schleppgas (insbesondere bei Prozessen unter aeroben Bedingungen), als auch mit beliebig elementar sauerstofffreien Gasen (insbesondere bei Prozessen unter anaeroben Bedingungen) durchgeführt werden.

Ein solches Aufarbeitungsverfahren bietet sich insbesondere dann an, wenn Aceton über einen Gasfermentationsprozess mit gasförmigen Substraten, insbesondere mit Kohlendioxid (CO₂),Kohlenmonoxid (CO), Synthesegas, Methan und Wasserstoff (H₂) oder beliebige Mischungen der zuvor genannten Gase hergestellt wird, da diese Gase dann nicht nur als Strippgas für das Aceton genutzt werden können, sondern gleichzeitig auch als Substrat (Feedgas) dienen. Somit können die zwei wichtigsten Arbeitsschritte (Fermentation & Aufarbeitung) elegant und ökonomisch miteinander verknüpft werden können und es auch innerhalb der beiden Arbeitsschritte zu keiner Kontamination durch ein organisches Lösungsmittel kommen, da jeweils mit Wasser gearbeitet wird.

Gegenstand der Erfindung ist somit ein Verfahren umfassend die Verfahrensschritte
A) Bereitstellen einer wässrigen Lösung enthaltend mindestens eine niedermolekulare, organische Verbindung ausgewählt aus der Gruppe Aceton, Isopropanol, Butanol und Ethanol, insbesondere Aceton, produzierende Mikroorganismen,
B) Einleiten mindestens eines Gases oder Gasgemisches in die wässrige Lösung,
C) Ausleiten des Gasstromes durch eine Wasser enthaltende Zusammensetzung ausgewählt aus der Gruppe Aceton, Isopropanol, Butanol und Ethanol, insbesondere Aceton, und gegebenenfalls
D) Abtrennen der niedermolekularen, organischen Verbindung von der Wasser enthaltenden Zusammensetzung.

Alle angegebenen Prozent (%) sind, wenn nicht anders angegeben, Massenprozent.
Wenn nicht anders angegeben, sind bezüglich der Umgebungsparameter wie Druck, Temperatur Standardbedingungen gemeint.

Die in dem erfindungsgemäßen Verfahren eingesetzten Mikroorganismen sind insbesondere Hefen und Bakterien. Insbesondere sind die Mikroorganismen ausgewählt aus Gattungen der Gruppe umfassend, bevorzugt bestehend aus, *Clostridium, Acetobacterium, Zymomonas, Escherichia,*

*Salmonella, Rhodococcus, Pseudomonas, Bacillus, Lactobacillus, Enterococcus, Alcaligenes, Klebsiella, Paenibacillus, Arthrobacter, Corynebacterium, Brevibacterium, Pichia, Candida, Hansenula und Saccharomyces.* Beispielhafte Arten dieser Gattungen sind *Escherichia coli, Alcaligenes eutrophus, Bacillus licheniformis, Paenibacillus macerans, Rhodococcus erythropolis, Pseudomonas putida, Enterococcus faecium, Enterococcus gallinarium, Enterococcus faecalis, Bacillus subtilis* und *Saccharomyces cerevisiae.*

In dem erfindungsgemäßen Verfahren wird bevorzugt ein Mikroorganismus, ausgewählt aus der Gruppe *Escherichia coli, Corynebacterium glutamicum, Clostridium spec., Clostridium aceticum, Acetobacterium woodii, Clostridium acetobutylicum, Clostridium beijerinckii, Yarrowia lipolytica, Saccharomyces spec., Saccharomyces cerevisiae* und *Pichia pastoris* eingesetzt.

Es ist erfindungsgemäß bevorzugt, dass es sich bei den Mikroorganismen um gentechnisch veränderte Mikroorganismen handelt. Insbesondere werden hier solche Mikroorganismen eingesetzt, die derart gentechnisch verändert wurden, als dass sie mehr niedermolekulare, organische Verbindungen zu produzieren vermögen, als ihr Wildtyp.

Solche gentechnisch veränderten Mikroorganismen sind für die Herstellung von Aceton als niedermolekulare, organische Verbindung in beispielsweise der EP2181195 beschrieben. Die in dieser Anmeldung offenbarten Zellen werden erfindungsgemäß bevorzugt in dem erfindungsgemäßen Verfahren zur Herstellung von Aceton eingesetzt. Solche Zellen sind insbesondere für Herstellungsverfahren unter aeroben Bedingungen geeignet.

Weitere solcher gentechnisch veränderten Mikroorganismen für die Herstellung von Aceton als niedermolekulare, organische Verbindung sind in der WO2010121849 beschrieben. Die in dieser Anmeldung offenbarten Zellen werden erfindungsgemäß bevorzugt in dem erfindungsgemäßen Verfahren zur Herstellung von Aceton eingesetzt, insbesondere bei Herstellungsverfahren unter anaeroben Bedingungen von Aceton.

Gentechnisch veränderte Mikroorganismen für die Herstellung von Butanol als niedermolekulare, organische Verbindung sind beispielsweise in der WO2008124523, der WO2008052973 und der WO2008052596 beschrieben. Die in dieser Anmeldung offenbarten Zellen werden erfindungsgemäß bevorzugt in dem erfindungsgemäßen Verfahren zur Herstellung von Butanol eingesetzt.

Gentechnisch veränderte Mikroorganismen für die Herstellung von Ethanol als niedermolekulare, organische Verbindung sind beispielsweise in der WO2011003893 und der WO2010130806 beschrieben. Die in dieser Anmeldung offenbarten Zellen werden erfindungsgemäß bevorzugt in dem erfindungsgemäßen Verfahren zur Herstellung von Ethanol eingesetzt.

In dem erfindungsgemäßen Verfahren kann in Verfahrensschritt B) jedes beliebige Gas oder Gasgemische eingesetzt werden, welches mit den niedermolekularen, organischen Verbindungen kein unerwünschten Nebenreaktionen eingeht oder toxisch für die eingesetzten Mikroorgansimen ist.

Bevorzugt enthält das Gas oder Gasgemisch mindestens eines ausgewählt aus der Gruppe umfassend, bevorzugt bestehend aus, Luft, Synthesegas, Stickstoff, Kohlenstoffdioxid, Kohlenstoffmonoxid, Wasserstoff, Sauerstoff und Methan.

Es ist erfindungsgemäß bevorzugt, als Gas oder Gasgemische in Verfahrensschritt B) ein elementaren Sauerstoff enthaltendes Gas oder Gasgemisch einzusetzen, wenn in Verfahrensschritt A) die Mikroorganismen die niedermolekularen, organischen Verbindungen unter aeroben Bedingungen produzieren. Unter dem Begriff "aerobe Bedingung" wird im Zusammenhang mit der vorliegenden Erfindung verstanden, dass ein Sauerstoffpartialdruck von > 0,01 bar vorliegt. In diesem Zusammenhang enthält das Gas oder Gasgemisch mindestens eines ausgewählt aus der Gruppe umfassend, bevorzugt bestehend aus Luft oder elementarem Sauerstoff.

Es ist erfindungsgemäß bevorzugt, als Gas oder Gasgemische in Verfahrensschritt B) ein Gas oder Gasgemisch einzusetzen, welches frei von elementarem Sauerstoff ist, wenn in Verfahrensschritt A) die Mikroorganismen die niedermolekularen, organischen Verbindungen unter anaeroben Bedingungen produzieren. Unter dem Begriff "anaerobe Bedingung" wird im Zusammenhang mit der vorliegenden Erfindung verstanden, dass ein Sauerstoffpartialdruck von ≤ 0,01 bar vorliegt. In diesem Zusammenhang enthält das Gas oder Gasgemisch bevorzugt mindestens eines ausgewählt aus der Gruppe umfassend, bevorzugt bestehend aus, Synthesegas, Stickstoff, CO₂, CO, H₂ und Methan.

In einer weiteren Ausführungsform des erfindungsgemäßen Verfahrens handelt es sich bei den in Verfahrensschritt A) eingesetzten Mikroorganismen um solche, die die klassische ABE-Fermentation in Verfahrensschritt A) durchführen; somit sind in diesem Zusammenhang die niedermolekularen, organischen Verbindungen Aceton, Ethanol und Butanol, insbesondere Aceton. Solche Organismen, die klassisch bei der ABE-Fermentation eingesetzt werden können, sind *Clostridium acetobutylicum* und *Clostridium beijerinckii,* welche sich unter strikt anaeroben Bedingungen vermehren und Mono-, Di- und Polysaccharide umsetzen.
Um das in dieser Verfahrensvariante in Verfahrensschritt B) eingesetzte Gas oder Gasgemisch handelt es sich bevorzugt um mindestens eines ausgewählt aus der Gruppe umfassend, bevorzugt bestehend aus, Stickstoff, Synthesegas, Kohlenstoffdioxid, Kohlenstoffmonoxid, H₂, Methan.

In einer weiteren bevorzugten Variante des erfindungsgemäßen Verfahrens handelt es sich bei den in Verfahrensschritt A) eingesetzten Mikroorganismen um acetogene Mikroorganismen, die Aceton als niedermolekulare, organische Verbindung aus mindestens einer Kohlenstoffquelle ausgewählt aus der Gruppe enthaltend Kohlendioxid und Kohlenmonoxid zu bilden vermögen und um das in Verfahrensschritt B) eingesetzte Gas oder Gasgemisch um mindestens eines ausgewählt aus der Gruppe umfassend, bevorzugt bestehend aus, Stickstoff, Synthesegas, Kohlenstoffdioxid, Kohlenstoffmonoxid, H₂. In dieser Verfahrensvariante bevorzugt eingesetzte Zellen sind in der WO 2010/121849 beschrieben.

Diese weitere, bevorzugte Variante des erfindungsgemäßen Verfahrens ist bevorzugt dadurch gekennzeichnet, dass die niedermolekulare, organische Verbindung Aceton ist, und als Mikroorganismen solche eingesetzt werden, die Aceton ausgehend von Acetyl-CoenzymA Aceton über
die enzymatische Umsetzung von Acetyl-CoA zu Acetoacetyl-CoA,
die enzymatische Umsetzung von Acetoacetyl-CoA zu Acetoacetat und CoA, wobei das
CoenzymA nicht auf ein Akzeptor-Molekül übertragen wird und
das Decarboxylieren von Acetoacetat zu Aceton und CO2
produzieren.

In Verfahrensschritt C) werden die niedermolekularen, organischen Verbindungen mindestens teilweise durch Ausleiten des Gasstromes aus dem wässrigen Fermentationsmedium durch eine Wasser enthaltende Zusammensetzung geleitet. Bevorzugt enthält die Wasser enthaltende Zusammensetzung mindestens 70 Gew.-% Wasser, bevorzugt mindestens 85 Gew.-% Wasser, besonders bevorzugt mindestens 90 Gew.-% Wasser, bezogen auf die Gesamtzusammensetzung.

Es ist vorteilhaft und bevorzugt, wenn die Wasser enthaltende Zusammensetzung gekühlt vorliegt. Hierdurch kann - bezogen auf die Wasser haltige Zusammensetzung - mehr niedermolekulare, organische Verbindung absorbiert werden. In diesem Zusammenhang bevorzugt Temperaturen, gemessen in der Wasser enthaltenden Zusammensetzung, liegen in einem Bereich von 1 °C bis 50° C, insbesondere von 5 °C bis 35 °C.

Das erfindungsgemäße Verfahren ist bevorzugt dadurch gekennzeichnet, dass der ausgeleitete Gasstrom in Verfahrensschritt C) durch mehrere, räumlich getrennte Wasser enthaltende Zusammensetzungen geleitet wird.

Es ist vorteilhaft und daher bevorzugt, wenn das die Wasser enthaltende Zusammensetzung verlassende und um niedermolekulare, organische Verbindung abgereicherte Gas oder Gasgemisch der wässrigen Lösung aus Verfahrensschritt A) wieder zugeführt wird, da das Absorbtionsmittel nicht toxisch für die Organismen ist und somit auch der abgereicherte Gasstrom sich nicht inhibierend auf den Fermentationsprozess auswirkt.

In Verfahrensschritt D) wird die Trennung der niedermolekulare, organische Verbindung, von der Wasser enthaltenden Zusammensetzung bevorzugt destillativ durch eine Druckveränderung und/oder durch eine Temperaturerhöhung (>20 °C bis <200 °C) erfolgen. So liegen beispielsweise die Siedepunkte bei Normaldruck für Aceton bei 56 °C, für Wasser bei 100 °C. Somit liegen in diesem Zusammenhang für ein System enthaltend Aceton und eine wässrige Zusammensetzung die bevorzugten Temperaturen in einem Bereich von 10 °C bis 100 °C, insbesondere von 35 °C bis 85 °C, um das Aceton von der Wasser enthaltenden Zusammensetzung zu trennen, wobei die Temperatur in der Wasser enthaltenden Zusammensetzung gemessen wird. In diesem Zusammenhang bevorzugte Drücke liegen in einem Bereich von 0 bis 3 bar (absolut), besonders bevorzugt ist in diesem Zusammenhang eine Kombination von Druck in einem Bereich von 0 bar bis Normaldruck (d.h. Umgebungsdruck) und einer Temperatur in Bereich von 35 bis 85 °C

Es ist erfindungsgemäß bevorzugt, wenn die Verfahrensschritte B) und C) zeitgleich mit der fermentativen Produktion von niedermolekularer, organischer Verbindung in Verfahrensschritt A) durchgeführt wird, so dass es sich um einen kontinuierlichen *in-situ*-Prozess handelt, bei dem das Produkt niedermolekulare, organische Verbindung während bzw. unmittelbar nach der Entstehung aus der Wasser enthaltenden Zusammensetzung ausgetragen wird.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll

Folgende Abbildungen sind Bestandteil der Beispiele:

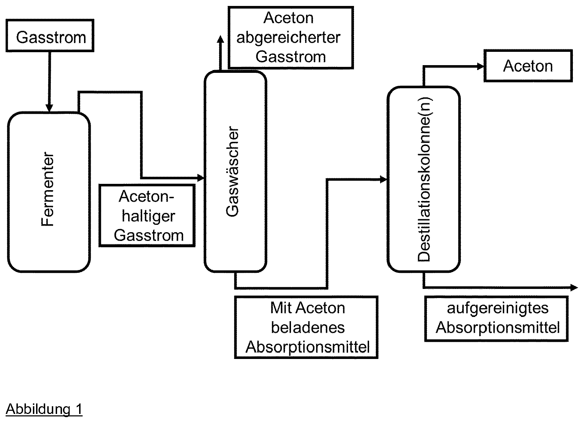

Abbildung 1: Abbildung 1 zeigt einen exemplarischen Aufarbeitungsprozess des erfindungsgemäßen Verfahrens Die Entfernung des Acetons aus der Fermentationsbrühe erfolgt durch Strippen mit einem Gas oder Gasgemisch noch während der Fermentation (*in-situ*); das Aceton wird aus dem Gasstrom mittels Gaswäsche mit Wasser (H₂O) als Absorptionsmittel durch Absorption gewonnen; Destillative Trennung des Aceton von evtl. Nebenkomponenten (z. B. Isopropanol, Ethanol, Butanol) und des Absorptionsmittels Wasser (H₂O), sowie Möglichkeit der Rückführung des aufgereinigten Absorptionsmittels in den Aufarbeitungsprozess vorne.
Abbildung 2: Stripverhalten von Aceton aus dem Fermentationsmedium unter Fermentationsbedingungen.
Abbildung 3: Absorptionsvermögen verschiedener Absorbentien für Aceton bei verschiedenen Absorptionsmittel/Aceton-Verhältnissen.
Abbildung 4: Verluste an Absorptionsmittel bei verschiedenen Absorptionsmittel/Aceton-Verhältnissen.

### Beispiele:

### Beispiel 1:

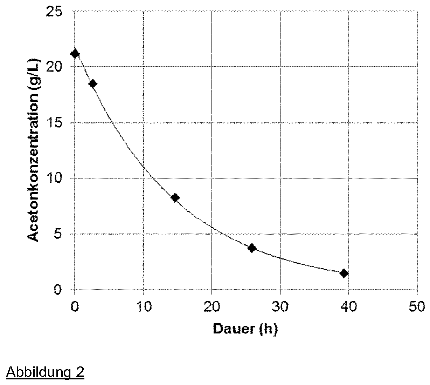

Schritt 1: Entfernung des Acetons aus der Fermentationsbrühe erfolgt durch Strippen mit einem Gas noch während der Fermentation (*in situ*)
   Im ersten Schritt muss Aceton aus einer wässrigen Fermenterlösung durch Begasung mit einem Gas ausgestrippt werden. Durch eine solche in-situ-Produktentfernung (Aceton) ist es möglich - unter der Vorrausetzung, dass inhibierende Produktkonzentrationen vermieden werden können - hohe Produktivitäten deutlich länger aufrechtzuerhalten.
   Aus Fermentationsversuchen mit acetogenen Bakterien ist bekannt, dass erst ab Acetonkonzentrationen von >30 g/L eine Limitierung des Wachstums zu beobachten ist.
   Um zu überprüfen, ob sich inhibierende Produktkonzentrationen in der Fermentationsbrühe durch das Ausstrippen verhindern lassen wurde die Kinetik des Ausstrippens von Acetons bestimmt. Für den Modellversuch wurde eine Aceton-Ausgangskonzentration von 22 g/L im Fermentationsmedium gewählt, diese liegt somit in derselben Größenordnung, in der bekannt ist, dass keine Inhibierung stattfindet. Das Fermentationsmedium wurde dabei 800 rpm gerührt und mit 0,5 Volumen Stripgas pro Volumen Reaktor pro Minute (vvm) begast. Als Stripgas wurde das Feedgas - das als Hauptbestandteile CO₂ und H₂ enthält - verwendet und die Temperatur der Fermenterlösung wurde auf 30°C eingestellt.
   Das Ergebnis ist in Abbildung 2 dargestellt. Es handelt sich bei der Abnahme der Aceton Konzentration um eine Reaktion erster Ordnung. Nach ∼10 h sind noch ∼50% der Anfangskonzentration im Fermentationsmedium, nach 21 h ∼25% Anfangskonzentration und nach 39h nur noch ∼10% der Aceton-Ausgangskonzentration erhalten. Daraus ableitend lässt sich für unterschiedliche Acetonproduktivitäten die sich einstellende Gleichgewichtskonzentrationen berechnen. Bei einer Acetonproduktivität von 0,3 gL⁻¹h⁻¹ liegt die Acetongleichgewichtskonzentration im Medium bei -4,5 g/L, erst ab einer Acetonproduktivität >2 gL⁻¹h⁻¹ ist mit einer Limitierung des Wachstums der acetogenen Bakterienstämmen zu erwarten, da dann die Acetongleichgewichtskonzentration im Medium bei > 30 g/L liegt.
   Somit ist die notwendige Bedingung für die erste Stufe des dreistufigen Aufarbeitungsprozesses, nämlich die Entfernung des Acetons durch Strippen mit einem Gas aus der Fermentationsbrühe während der Fermentation (*in situ*), erfüllt. Das mit Aceton angereicherte Strippgasstrom wird dann in Schritt 2 über einen Gaswäscher geleitet und mit Hilfe von Wasser (H₂O) als Absorptionsmittel absorbiert.
Schritt 2: Aceton wird mittels Gaswäsche aus dem Gasstrom durch Absorption gewonnen mit Wasser (H₂O) als Absorptionsmittel.

Die Gewinnung des Acetons aus dem Stripgasstrom erfolgt durch Absorption in einem Gaswäscher. Für die Beurteilung der Eignung wurden zunächst verschiedene Absorptionsmittel betrachtet. Hierfür wurden neben dem Absorptionsvermögen bezüglich Aceton auch die Verluste an Absorptionsmittel im Stripgas betrachtet. Neben dem erfindungsgemäßen Absorbens Wasser, wurden auch die organischen Absorptionsmittel Isophoron, N-Methyl-2-pyrrolidon (NMP), Ethanol, Isopropanol und Toluol getestet.

Für die Experimente wurde ein kontinuierlicher Aceton-haltiger Stripgasstrom bestehend aus 8,2 Gew.-% Aceton mit einem Gasgemisch bestehend aus CO₂, H₂, N₂ und Methan erzeugt und über die genannten Absorbentien geleitet. Die genaue Zusammensetzung des Stripgasstroms ist der nachfolgenden Tabelle 1 zu entnehmen:

**Tabelle 1: Zusammensetzung Aceton-haltiger Stripgasstrom**

| Zusammensetzung Strippgasstrom | Massenfraktion (%) |
|---|---|
| H₂ | 0,7 |
| CO₂ | 85,0 |
| N₂ | 1,8 |
| Aceton | 8,2 |
| Ethanol | 0,2 |
| Wasser | 2,8 |

Der Stripgasstrom hatte eine Temperatur von 35°C und die Absorption wurde unter Umgebungsdruck (1 bar) durchgeführt. Das Verhältnis der eingesetzten Absorptionsmittel zum ankommenden Massestrom an Aceton wurde variiert im Bereich von 6,6 bis 19,5 (Massen %).

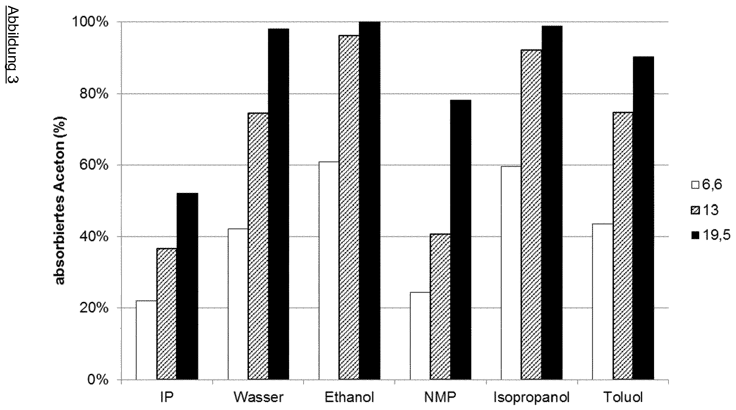

Die Ergebnisse sind in der nachfolgenden Abbildung 3 dargestellt. Für alle untersuchten Absorbentien gilt, je höher der Überschuss an Absorbens, desto besser ist die Wiederfindungsrate von Aceton. Hierbei zeigen unter den getesteten Absorbentien Wasser, Ethanol und Isopropanol sehr gute Ergebnisse. Bei einem Absorptionsmittel/Aceton-Verhältnis von 19,5 zeigen diese Absorbentien eine nahezu quantitative Absorption (>98%) des Acetons aus dem Aceton-haltigen Stripgasstrom.

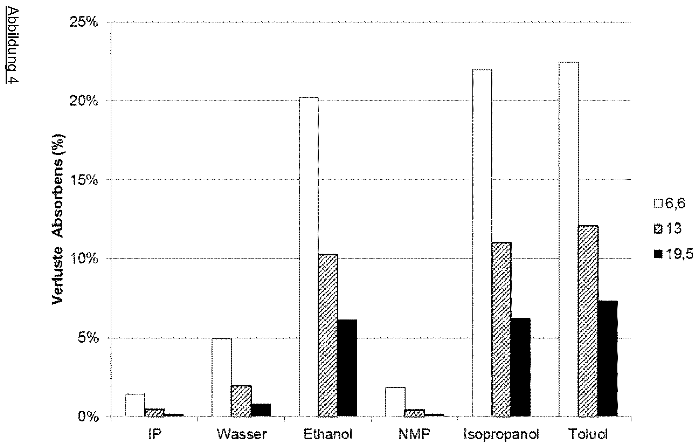

Neben dem Kriterium der Wiederfindungsrate des Acetons, also wieviel des Aceton absorbiert wird ist auch entscheidend, wie effizient das eingesetzte Absorptionsmittel genutzt werden kann. Nachfolgend (Abbildung 4) sind die Verluste an Absorptionsmittel, die im Gaswäscher über Kopf verloren gehen, dargestellt. Hier zeigt sich, dass die beiden Absorbentien Ethanol und Isopropanol - die jeweils Aceton nahezu quantitativ absorbiert haben - relativ hohe Verluste über Kopf am Gaswäscher zeigen. Im Fall von Ethanol gehen bis zu 20%, im Fall von Isopropanol sogar bis zu 22% verloren. Bei Wasser (H₂O) hingegen ist die Verlustrate in allen betrachteten Fällen gerade einmal im Bereich < 5%, bei einem H₂O/Aceton-Verhältnis von 19,5 sogar < 1%.

Innerhalb der untersuchten Absorptionsmittel Isophoron, Wasser, NMP Ethanol, Isopropanol und Toluol zeigt Wasser in Summe die besten Eigenschaften. Das Absorptionsvermögen von Aceton in Wasser mittels Gaswäsche ist dabei nahezu quantitativ (>98%) und gleichzeitig ist der Verlust des Absorbens Wasser gering (<1%). Im Gegensatz zu organischen Substanzen als Absorptionsmittel ist Wasser extrem kostengünstig und im Vergleich zum Stand der Technik wird also kein organisches Lösungsmittel für die Absorption benötigt, sowohl die Fermentation, als auch die Aufarbeitung können im wässrigen Milieu durchgeführt werden.

Schritt 3: Destillative Trennung von Aceton und dem Absorptionsmittel Wasser, sowie Möglichkeit der Rückführung des aufgereinigten Absorptionsmittels in den Aufarbeitungsprozess (Schritt 2).

Aceton lässt sich aufgrund seines geringen Siedepunkts von 56°C sehr einfach vom Wasser destillativ trennen. Für das Reaktionsgemisch Wasser/Aceton liegt unter Normaldruck kein Azeotrop vor und es kann somit in hoher Reinheit gewonnen werden. Neben der Trennung von Aceton und des Absorptionsmittels (Wasser) kann auch die Abtrennung weiterer Nebenkomponenten wie Ethanol und Isopropanol berücksichtigt werden.

In einer zweistufigen Destillation des Reaktionsgemisches aus Schritt 2 bestehend hauptsächlich aus Wasser und Aceton wurde eine sehr gute Trennung von Aceton und Wasser, sowie den weiteren Komponenten (u.a. Ethanol, Isopropanol) erreicht wird. Die Destillationen wurde bei Normaldruck (1 bar) und bei einer Temperaturen von 80°C im Sumpf durchgeführt. Das so erhaltene Aceton erfüllt dabei alle Anforderungen an seine Reinheit für eine chemische Weiterverwendung, die Reinheit des Acetons beträgt unter den gegebenen Bedingungen > 97%. Gleichzeitig besteht die Möglichkeit der Rückführung des aufgereinigten Absorptionsmittels (Reinheit >99%) in den Aufarbeitungsprozess (Schritt 2).
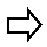 Der entwickelte drei-stufige Aufarbeitungsprozess mit Wasser als Absorptionsmittel hat über die drei Stufen eine Gesamtausbeute von mindestens 95%, ist somit kostengünstig, gut skalierbar und im Fermentationsprozess kann durch die in-situ Abtrennung eine Inhibierung der Produktbildung durch hohe Acetonkonzentrationen vermieden werden. Gleichzeitig wird durch die Verwendung des Absorptionsmittels Wasser eine nahezu quantitative Wiederfindungsrate von Aceton erreicht und somit eine sehr gute Gesamtausbeute des Aufarbeitungsprozesses erreicht.

### Beispiel 2 (nicht erfindungsgemäß)

Wird der drei-stufige Aufarbeitungsprozess unter identischen Bedingungen wie im erfindungsgemäßen Beispiel 1 mit Isophoron anstelle von Wasser als Absorbtionsmittel (Schritt 2) durchgeführt, reduziert sich die Gesamtausbeute von >95% auf bestenfalls ∼51% (Isophoron/Aceton-Verhältnis von 19,5 im Gaswäscher) aufgrund des geringeren Absorptionsvermögens. Reduziert man das Isophoron/Aceton-Verhältnis auf 6,6 Im Gaswäscher sinkt die Gesamtausbeute auf nur noch ∼21%.

## Patentansprüche

1. Verfahren umfassend die Verfahrensschritte
A) Bereitstellen einer wässrigen Lösung enthaltend mindestens eine niedermolekulare, organische Verbindung ausgewählt aus der Gruppe Aceton, Isopropanol, Butanol und Ethanol produzierende Mikroorganismen,
B) Einleiten mindestens eines Gases oder Gasgemisches in die wässrige Lösung,
C) Ausleiten des Gasstromes durch eine Wasser enthaltende Zusammensetzung und gegebenenfalls
D) Abtrennen der niedermolekularen, organischen Verbindung von der Wasser enthaltenden Zusammensetzung.

2. Verfahren gemäß Anspruch 1, **dadurch gekennzeichnet, dass** die Mikroorganismen ausgewählt sind aus Gattungen der Gruppe umfassend, bevorzugt bestehend aus, *Clostridium, Acetobacterium, Zymomonas, Escherichia, Salmonella, Rhodococcus, Pseudomonas, Bacillus, Lactobacillus, Enterococcus, Alcaligenes, Klebsiella, Paenibacillus, Arthrobacter, Corynebacterium, Brevibacterium, Pichia, Candida, Hansenula* und *Saccharomyces.*

3. Verfahren gemäß mindestens einem der vorherigen Ansprüche, **dadurch gekennzeichnet, dass** das Gas oder Gasgemisch mindestens eines enthält ausgewählt ist aus der Gruppe umfassend Luft, Synthesegas, Stickstoff, Kohlenstoffdioxid, Kohlenstoffmonoxid, Wasserstoff, Sauerstoff und Methan.

4. Verfahren gemäß mindestens einem der vorherigen Ansprüche, **dadurch gekennzeichnet, dass** *Clostridium spec., Clostridium aceticum, Acetobacterium woodii, Clostridium acetobutylicum* und *Clostridium beijerinckii,* insbesondere *Clostridium acetobutylicum* und *Clostridium beijerinckii,* als Mikroorganismen und Gas oder Gasgemische ausgewählt aus der Gruppe umfassend, bevorzugt bestehend aus, Stickstoff, Synthesegas, Kohlenstoffdioxid, Kohlenstoffmonoxid, H₂, Methan eingesetzt werden.

5. Verfahren gemäß mindestens einem der vorherigen Ansprüche, **dadurch gekennzeichnet, dass** die niedermolekulare, organische Verbindung Aceton ist, und als Mikroorganismen solche eingesetzt werden, die Aceton ausgehend von Acetyl-CoenzymA Aceton über
die enzymatische Umsetzung von Acetyl-CoA zu Acetoacetyl-CoA,
die enzymatische Umsetzung von Acetoacetyl-CoA zu Acetoacetat und CoA, wobei das CoenzymA nicht auf ein Akzeptor-Molekül übertragen wird und
das Decarboxylieren von Acetoacetat zu Aceton und CO₂
produzieren.

6. Verfahren gemäß mindestens einem der vorherigen Ansprüche, **dadurch gekennzeichnet,**
**dass** die niedermolekulare, organische Verbindung Aceton ist, acetogene Mikroorganismen, die Aceton aus mindestens einer Kohlenstoffquelle ausgewählt aus der Gruppe enthaltend Kohlendioxid und Kohlenmonoxid zu bilden vermögen, eingesetzt werden, und das in Verfahrensschritt B) eingesetzte Gas oder Gasgemisch mindestens eines ausgewählt aus der Gruppe umfassend Stickstoff, Synthesegas, Kohlenstoffdioxid, Kohlenstoffmonoxid, H₂ ist.

7. Verfahren gemäß mindestens einem der vorherigen Ansprüche, **dadurch gekennzeichnet,**
**dass** der ausgeleitete Gasstrom in Verfahrensschritt C) durch mehrere, räumlich getrennte Wasser enthaltende Zusammensetzungen geleitet wird.

8. Verfahren gemäß mindestens einem der vorherigen Ansprüche, **dadurch gekennzeichnet,**
**dass** das die Wasser enthaltende Zusammensetzung verlassende Gas oder Gasgemisch der wässrigen Lösung aus Verfahrensschritt A) wieder zugeführt wird.

9. Verfahren gemäß mindestens einem der vorherigen Ansprüche, **dadurch gekennzeichnet,**
**dass** in Verfahrensschritt A) die Mikroorganismen die niedermolekularen, organischen Verbindungen unter aeroben Bedingungen produzieren und als Gas oder Gasgemische in Verfahrensschritt B) ein elementaren Sauerstoff enthaltendes Gas oder Gasgemisch eingesetzt wird.

10. Verfahren gemäß mindestens einem der vorherigen Ansprüche, **dadurch gekennzeichnet,**
**dass** in Verfahrensschritt A) die Mikroorganismen die niedermolekularen, organischen Verbindungen unter anaeroben Bedingungen produzieren und Verfahrensschritt B) ein Gas oder Gasgemisch, welches frei von elementarem Sauerstoff ist, eingesetzt wird.
